(19) Europäisches Patentamt
European Patent Office
Office européen des brevets

(11) Publication number: 0 168 841 B1

(12) # EUROPEAN PATENT SPECIFICATION

(45) Date of publication of patent specification: 28.09.88

(51) Int. Cl.[4]: C 07 D 211/90

(21) Application number: 85109347.6

(22) Date of filing: 19.12.83

(60) Publication number of the earlier application in accordance with Art. 76 EPC: 0 116 769

(54) **Dihydropyridine intermediate.**

(43) Date of publication of application: 22.01.86 Bulletin 86/04

(45) Publication of the grant of the patent: 28.09.88 Bulletin 88/39

(84) Designated Contracting States: BE CH DE FR GB IT LI LU NL SE

(58) References cited:
EP-A-0 060 674
EP-A-0 089 167

DERWENT JAPANESE PATENTS, Chemical Section, Derwent Publications Ltd., vol. 4, no. 85 (C-15)567r, 18th June 1980; & JP - A - 55 47 656 (DAINIPPON SEIYAKU K.K.) 04-04-1980

(73) Proprietor: Pfizer Limited
Ramsgate Road
Sandwich Kent CT13 9NJ (GB)
(84) GB

(73) Proprietor: Pfizer Corporation
Calle 15 1/2 Avenida Santa Isabel
Colon (PA)
(84) BE CH DE FR IT LI LU NL SE

(72) Inventor: Campbell, Simon Fraser, Dr.
Grey Friars Upper Street
Kingsdown Deal Kent (GB)
Inventor: Cross, Peter Edward, Dr.
21, Cherry Avenue
Canterbury Kent (GB)
Inventor: Stubbs, John Kendrick, Dr.
111, Blenheim Road
Deal Kent (GB)
Inventor: Arrowsmith, John Edmund, Dr.
340, Dover Road
Upper Walmer Deal Kent (GB)

(74) Representative: Wood, David John et al
Pfizer Limited Ramsgate Road
Sandwich Kent CT13 9NJ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 168 841 B1

**Description**

This application is a divisional from EP—A—0116769 (83307719.1), and concerns the compound 2-(2-aminoethoxymethyl)-4-(2-chloro-3-trifluoromethylphenyl)-3-ethoxycarbonyl-5-methoxycarbonyl-6-methyl-1,4-dihydropyridine.

This compound is a useful synthetic intermediate as described in the said copending application. It is also clear from our copending EP—A—0116769 that this compound serves as a starting material for a class having utility as anti-ischaemic and antihypertensive agents, cf. EP—B1—0116769, p 56 and 57.

The compound can be prepared as described in the following Examples and Preparations.

Example 1

Preparation of 2-(2-aminoethoxymethyl)-4-(2-chloro-3-trifluoromethylphenyl)-3-ethoxycarbonyl-5-methoxycarbonyl-6-methyl-1,4-dihydropyridine

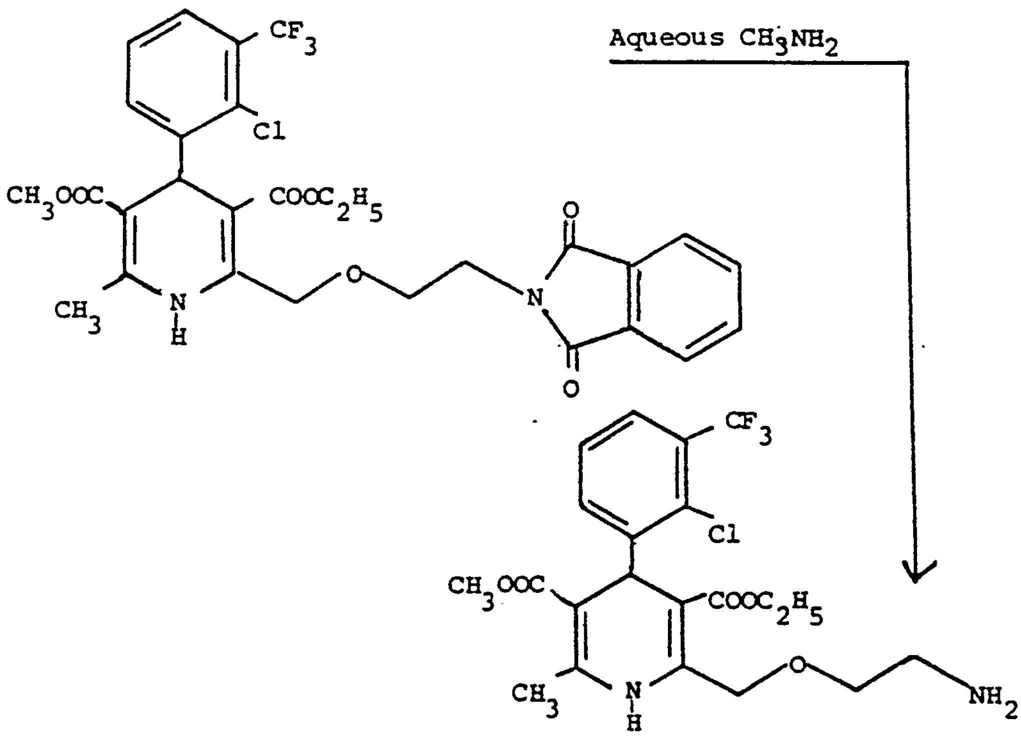


The phthalimido compound from Preparation 1 (2.8 g) was added to aqueous methylamine (14 ml of 40%) and stirred at room temperature for 17 hours. The resultant solid was filtered, redissolved in chloroform (50 ml), dried ($MgSO_4$), filtered and evaporated to give a yellow solid. Crystallisation from hexane gave the title compound, yield 1.0 g, m.p. 122°C.

Analysis:

| | | | |
|---|---|---|---|
| Found: | C, 53.25; | H, 4.9; | N, 5.75%; |
| Calculated from $C_{21}H_{24}ClF_3N_2O_5$: | C, 52.9; | H, 5.1; | N, 5.9%. |

Example 2

Preparation of 2-[2-aminoethoxymethyl]-4-(2-chloro-3-trifluoromethylphenyl)-3-ethoxycarbonyl-5-methoxycarbonyl-6-methyl-1,4-dihydropyridine

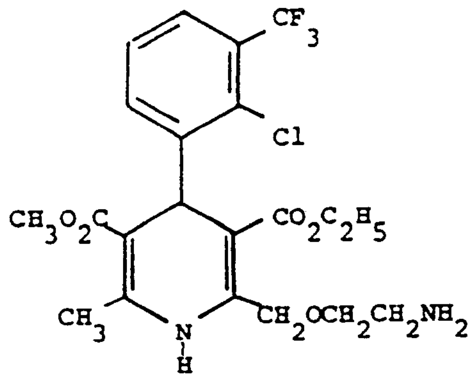

The title compound was prepared by the catalytic hydrogenation of the azido compound of Preparation 2 by the method described in Preparation 1 of the said copending application EP—A—0116769. This compound was confirmed by n.m.r. and i.r. analysis to be identical to the product of Example 1 above.

The following preparations describe the preparation of certain starting materials.

Preparation 1

The following compound, m.p. 179°C was prepared similarly to Preparation 5 of the said copending application EP—A—0116769 but using 2-chloro-3-trifluoromethylbenzaldehyde in stage (ii). The reaction time was the same.

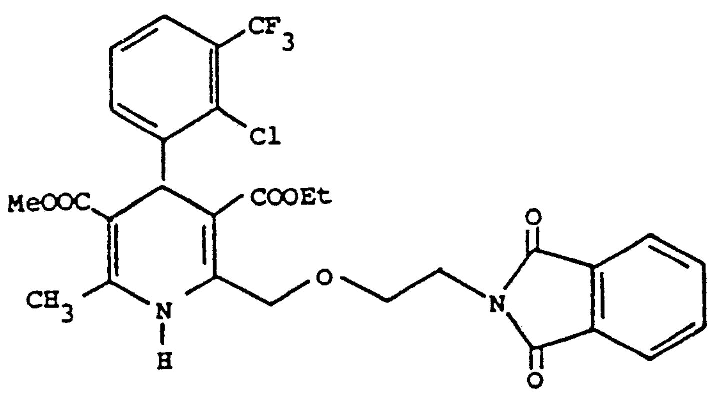

Analysis:

| | | | |
|---|---|---|---|
| Found: | C, 57.2; | H, 4.45; | N, 4.8%; |
| Calculated for $C_{29}H_{26}ClF_3N_2O_7$: | C, 57.4; | H, 4.3; | N, 4.6%. |

Preparation 2

Preparation of 2-(2-azidoethoxymethyl)-4-(2-chloro-3-trifluoromethylphenyl)-3-ethoxycarbonyl-5-methoxycarbonyl-6-methyl-1,4-dihydropyridine

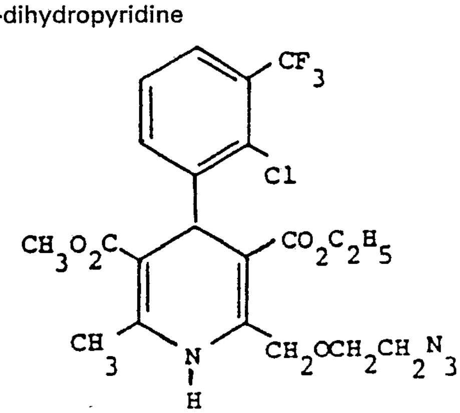

The title compound, m.p. 143—145°C, was prepared similarly to the method described in Preparation 3 of the said copending application EP—A—0116769 but using 2-chloro-3-trifluoromethylbenzaldehyde. The reaction time was the same.

Analysis:

| | | | |
|---|---|---|---|
| Found: | C, 50.2; | H, 4.4; | N, 11.3%; |
| Calculated for $C_{21}H_{22}ClF_3N_4O_5$: | C, 50.15; | H, 4.4; | N, 11.1%. |

Preparation 3

Preparation of 2-chloro-3-trifluoromethylbenzaldehyde

2-Chloro-1-trifluoromethylbenzene (54.15 g) was dissolved in dry tetrahydrofuran (500 ml) and stirred while cooling to −68°C under a stream of dry nitrogen. (The whole reaction is carried out under dry nitrogen until the additon of distilled water.) To this was added n-butyl lithium (180 ml of 1.6 M solution in hexane) dropwise keeping the temperature below −60°C. After stirring at −68°C for a further 2 hours, a solution of dimethylformamide (22 ml) in dry tetrahydrofuran (100 ml) was added dropwise keeping the temperature below −60°C. The reaction mixture was allowed to warm to room temperature slowly over 17 hours and distilled water (200 ml) was then added. The organic phase was separated off and the aqueous liquors were extracted with ether (100 ml). The combined ether extracts plus the organic phase were washed with saturated brine, dried ($MgSO_4$), filtered and evaporated to give 61.5 g of an orange oil, being the crude title compound.

This oil was then added to an aqueous sodium bisulphite solution (65 g in 600 ml distilled water) and heated at 60°C for 0.5 hours. The solution was extracted with methylene chloride (3 × 100 ml) and, after acidification of the aqueous phase with concentrated sulphuric acid to pH=1, was heated to 100°C for a further 0.5 hours. The resultant aqueous solution was extracted with methylene chloride (3 × 200 ml) and the combined organic extracts were dried ($MgSO_4$), filtered and evaporated to give 42 g of a colourless solid which was crystallised from hexane to give the *title compound*, m.p. 43—44°C.

Analysis:

| | | |
|---|---|---|
| Found: | C, 45.9; | H, 2.0%; |
| Calculated for $C_8H_4F_3C10$: | C, 46.1; | H, 2.0%. |

**Claim**

2-(2-aminoethoxymethyl)-4-(2-chloro-3-trifluoromethylphenyl)-3-ethoxycarbonyl-5-methoxycarbonyl-6-methyl-1,4-dihydropyridine.

**Patentanspruch**

2-(2-Aminoethoxymethyl)-4-(2-chloro-3-trifluoromethylphenyl)-3-ethoxycarbonyl-5-methoxycarbonyl-6-methyl-1,4-dihydropyridin.

**Revendication**

2-(2-aminoéthoxyméthyl)-4-(2-chloro-3-trifluorométhylphényl)-3-éthoxycarbonyl-5-méthoxycarbonyl-6-méthyl-1,4-dihydropyridine.